Europäisches Patentamt

European Patent Office

Office européen des brevets

(11) Veröffentlichungsnummer: **0 610 842 A2**

(12) **EUROPÄISCHE PATENTANMELDUNG**

(21) Anmeldenummer: **94101794.9**

(22) Anmeldetag: **07.02.94**

(51) Int. Cl.5: **C12N 15/31**, C07K 13/00,
C12N 15/80, C12N 1/15,
//(C12N1/15,C12R1:645)

(30) Priorität: **12.02.93 DE 4304312**

(43) Veröffentlichungstag der Anmeldung:
**17.08.94 Patentblatt 94/33**

(84) Benannte Vertragsstaaten:
**BE DE DK ES FR GB GR IE IT LU NL PT**

(71) Anmelder: **HOECHST AKTIENGESELLSCHAFT
Brüningstrasse 50
D-65929 Frankfurt am Main (DE)**

(72) Erfinder: **Kück, Ulrich, Dr.
Vorm Felde 11
D-44797 Bochum (DE)**
Erfinder: **Nowak, Claudia
Am Trienensiepen 2
D-44229 Dortmund (DE)**
Erfinder: **Walz, Markus, Dr.
Leifacker 1
D-44892 Bochum (DE)**

(54) Beta-Tubulin von Acremonium chrysogenum, seine Herstellung und Verwendung.

(57) Die Erfindung betrifft $\beta$-Tubulin von Acremonium chrysogenum (A. chrysogenum), seine Aminosäuresequenz und die Verwendung des Gens für die Transformation von A. chrysogenum.

Fig. 1

Die Erfindung betrifft β-Tubulin von Acremonium chrysogenum (A. chrysogenum), seine Gen- und Aminosäuresequenz und die Verwendung für die Transformation von A. chrysogenum.

Bei Eukaryonten existieren drei verschiedene Typen von Tubulin-Genen und zwar für Alpha-, Beta- und Gamma-Tubuline. Die Tubulin-Gene kodieren für eine Familie von Tubulin-Proteinen, die eine wichtige Rolle im Zellzyklus bei Eukaryonten spielen. So können z. B. Mutationen in den Tubulin-Polypeptiden auf die Bildung der Mikrotubuli wirken. Auch können die Mutationen die Empfindlichkeit gegenüber Tubulin-Inhibitoren verändern. So wirkt Benomyl, ein potentes Fungizid, spezifisch auf das β-Tubulin.

Die molekulare Analyse der β-Tubulin-Gene und die Untersuchung der Benomyl-resistenten Mutanten hat gezeigt, daß vier individuelle Punktmutationen im β-Tubulin-Gen, bei denen es sich in allen Fällen um Substitutionen handelt, zur Resistenz gegenüber dem Fungizid Benomyl führen können (Zusammenfassung bei Osmani und Oakley (1991) Cell Cycle and Tubulin Mutations in Filamentous Fungi. In J. W. Bennett and L. L. Lasure (eds) "More Gene Manipulations in Fungi". pp. 107-122. Academic Press San Diego). Die ersten mutierten β-Tubulin-Gene wurden von Aspergillus nidulans-Stämmen, die Resistenz gegenüber Benomyl zeigen, isoliert. In einigen Fällen wurden die mutierten β-Tubulin-Gene auch zur Transformation von z. B. Neurospora crassa (Orbach M. J., Porro E. B., Yanofsky C. (1986) Mol. Cell. Biol. 6: 231-243) oder Aspergillus nidulans (May GS et al. (1987) Gene 55: 231-343) verwendet.

Das mutierte β-Tubulin-Gen von Neurospora crassa wurde nicht nur zur erfolgreichen Transformation innerhalb derselben Art verwendet, sondern konnte auch genutzt werden, um andere Pilze erfolgreich zu transformieren (Osmani und Oakley (1991) Cell Cycle and Tubulin Mutations in Filamentous Fungi. In J. W. Bennett and L. L. Lasure (eds) "More Gene Manipulations in Fungi". pp. 107-122. Academic Press, San Diego). Die transformierten Pilze können im Vergleich zu Kontrollstämmen an ihrer Benomyl-Resistenz erkannt werden.

Für A. chysogenum wurde bisher nur ein einziges homologes Transformationssystem beschrieben. Hierbei wird das Nitratreduktasekodierende Gen niaD eingesetzt. Allerdings ist als Rezipientenstamm eine niaD-Mutante notwendig, um die Transformation erfolgreich durchführen zu können.

Man stellte sich nun die Aufgabe, ein Transformationssystem für A. chrysogenum zu finden, das nicht auf einen Rezipientenstamm beschränkt ist.

Überraschenderweise wurde nun gefunden, daß sich das β-Tubulin-Gen aus A. chrysogenum für die Transformation von beliebig vielen A. chrysogenum-Stämmen eignet, sei es Wildtypstämme oder Mutanten davon.

Ein Gegenstand der vorliegenden Erfindung ist daher das β-Tubulin von A. chrysogenum, insbesondere mit der Aminosäuresequenz gemäß Tab. 2 A und B.

Ein weiterer Gegenstand ist die DNA, die für β-Tubulin oder Teile davon kodiert, insbesondere mit der DNA-Sequenz gemäß Tab. 2 A und B, vor allem die DNA, die für den proteinkodierenden Teil von β-Tubulin kodiert.

Ferner ist ein Gegenstand der Erfindung eine DNA, die unter stringenten Bedingungen, insbesondere bei 68°C in 6x SSC-Puffer (NaCl-Citratpuffer) zu dem proteinkodierten Bereich der DNA-Sequenz gemäß Tab. 2 A und B oder Teile davon hybridisiert. Vor allem ist eine DNA Gegenstand der Erfindung, die an der Stelle 167 der Aminosäuresequenz gemäß Tab. 2 A für Tyrosin kodiert und somit eine künstliche Mutante darstellt. Die künstliche Mutante erwies sich als besonders vorteilhaft bei der Transformation von A. chrysogenum.

Darüberhinaus sind Gegenstände der Erfindung Vektoren und Wirtszellen, die die für β-Tubulin-kodierende DNA oder Teile davon enthalten und außerdem der Vektor pAUL1. Dieser Vektor ist besonders vorteilhaft bei der Biosynthese von Cephalosporin, da er kein β-Lactamasegen enthält, das bei Expression die Ausbeuten an Cephalosporin verringern kann.

Weitere Gegenstände der Erfindung sind Verfahren zur Herstellung von β-Tubulin und Transformation von A. chrysogenum.

Das gentechnische Verfahren zur Herstellung von β-Tubulin erfolgt nach dem Fachmann allgemein bekannten Verfahren. Als Expressionsvektor eignet sich jeder bekannte Vektor und als Wirtszelle eignet sich jede pro- oder eukaryotische Wirtszelle einschließlich Pilze, wie z. B. E. coli, CHO-, BHK-Zellen oder Hefen.

Bei der Transformation von A. chrysogenum werden vorzugsweise zuerst Protoplasten nach dem Fachmann allgemein bekannten Methoden aus A. chrysogenum hergestellt, diese werden dann mit für β-Tubulin-kodierender DNA oder Vektoren dafür in Kontakt gebracht und anschließend in einem geeigneten Medium regeneriert. Hierbei wurde überraschenderweise gefunden, daß die Regenerationsrate der Protoplasten durch die Verwendung von Medien mit einer Saccharosekonzentration von 11 bis 50 Gew.-%, insbesondere von 11 bis 30 Gew-%, vor allem 15 bis 25 Gew.-% deutlich gesteigert werden konnte.

Das erfindungsgemäße Transformationsverfahren kann auch besonders vorteilhaft bei der Kotransformation mit anderen Vektoren eingesetzt werden, die beliebige Fremdgene enthalten. Daher kann A. chrysoge-

EP 0 610 842 A2

num als Wirtszelle besonders erfolgreich bei der Herstellung von beliebigen Fremdproteinen, wie z. B. die Glutarylacylase, die für die Biosynthese von Cephalosporin verwendet wird, eingesetzt werden. Auch dient das erfindungsgemäße homologe Transformationssystem für A. chrysogenum im Hinblick auf Arbeitsbeschränkungen für gentechnische Arbeiten in verschiedenen Ländern als biologische Sicherheitsmaßnahme.

Die Erfindung wird nun anhand der Figuren und Beispiele näher erläutert, ohne darauf beschränkt zu sein. Die beispielhaft erläuterten Experimentalschritte werden im folgenden aufgelistet:

1) Isolierung des $\beta$-Tubulin-Gens von A. chrysogenum

2) DNA-Sequenzierung des $\beta$-Tubulin-Gens

3) In vitro-Mutagenese des $\beta$-Tubulin-Gens

4) Transformation von A. chrysogenum mit dem mutierten $\beta$-Tubulin-Gen

5) Konstruktion eines Transformations-Vektors pAUL1, der kein $\beta$-Lactamase-Gen enthält

6) Kotransformation von A. chrysogenum unter Verwendung des mutierten $\beta$-Tubulin-Gens

Die Tabellen und Figuren sind wie folgt beschrieben:

| | |
|---|---|
| Tab. 1 | Sequenz der verwendeten Oligonukleotide für die DNA-Sequenzierung und in vitro-Mutagenese. Das Oligonukleotid Nr. 375 wurde für die in vitro-Mutagenese, die Oligonukleotide Nr. 341, 375, 376 wurden zur Amplifikation von DNA-Fragmenten mit der PCR-Methode verwendet. Alle übrigen Oligonukleotide fanden als Startermoleküle bei der DNA-Sequenzierung Verwendung. |
| Tab. 2 Teil A und B: | DNA-Sequenz des $\beta$-Tubulin-Genbereichs. Die abgeleitete Aminosäuresequenz der Exonen ist wiedergegeben. In den Intronen wurden für pilzliche Eukaryonten typische Konsensus-Sequenzen unterstrichen. |
| Tab. 3: | Vergleich der Intron-Konsensus-Sequenzen verschiedener pilzlicher Gene. Abkürzungen: IVS1-4, Intron1-4; N.c., Neurospora crassa; A.n., Aspergillus nidulans; S.c., Saccharomyces cerevisiae; h.e., höhere Eukaryonten. |
| Tab. 4: | Vergleich der $\beta$-Tubulin-Sequenzen verschiedener Eukaryonten. Die Ähnlichkeitswerte (%) beziehen sich auf die A. chrysogenum-Sequenz. Referenzen der verwendeten Sequenzen: |
| | 1) diese Arbeit |
| | 2) Orbach M. J. et al. (1986) |
| | 3) May G. S. et al. (1987) |
| | 4) Neff N. F. et al. (1983) Cell 33: 211-219 |
| | 5) Youngblom J. et al. (1984) Mol. Cell. Biol. 4: 2686-2696 |
| | 6) Silflow C. D. et al. (1987) Devel. Genet. 8: 435-460 |
| | 7) Valenzuela P. et al. (1981) Nature 289: 650-655 |
| Tab. 5: | Sequenz des Oligonukleotids 375, das für die in vitro-Mutagenese verwendet wurde und die davon abgeleitete Aminosäuresequenz. Als Vergleich ist die Wildtypsequenz angegeben. |
| Fig. 1: | Restriktionskarte des rekombinanten Klon L-T6 mit dem $\beta$-Tubulin-Gen von A. chrysogenum (schraffiert). Der Pfeil gibt die Transkriptionsrichtung des $\beta$-Tubulin-Gens an. Vergrößert wurde der sequenzierte Teil dargestellt, die Exon- (schwarz) und die Intron- (weiß) Bereiche sind gekennzeichnet. Die Pfeile stellen die ermittelten Teilsequenzen dar und dokumentieren die Sequenzierungsstrategie. Abkürzungen: B, BamHI; Ba, BalI; E, EcoRI; H, HinfIII; K, KpnI; M, MluI; Ms, MstI; N, NcoI; P, PvuII; S, SalI; Sn, SnaI; Sp, SphI; St, StuI; X, XbaI; Xm, XmaIII |
| Fig. 2: | Restriktionskarte des Vektors pCN3, der zur Transformation von Acremonium chrysogenum geeignet ist. Aufgrund der Nukleotid-Substitution von T nach A (T → A) wurde das Aminosäure-Nukleotid des Wildtyp-$\beta$-Tubulin-Gens (schraffiert) in vitro mutiert. Auf dem Plasmid sind außerdem das bakterielle Gen für eine $\beta$-Lactamase (Amp^R) und das Gen für eine $\beta$-Galactosidase (LacZ) enthalten. Außerdem wurden repräsentative Restriktionsschnitte für die folgenden Endonukleasen innerhalb des $\beta$-Tubulin-Gens gekennzeichnet: BalI, Ba; EcoRI, E; PvuI; SacI; SphI, Sp; XbaI, X. Der Teil des Plasmids, welcher von Acremonium chrysogenum stammt, befindet sich zwischen den Schnittstellen für die Enzyme EcoRI und XbaI und wurde durch eine doppelte Linie gekennzeichnet. |
| Fig. 3: | Repräsentative Autoradiogramme von DNA-Sequenzierungen, bei denen PCR-amplifizierte $\beta$-Tublin-Genfragmente direkt sequenziert wurden. Die Sequenz einer Transformante (T) wurde im Vergleich zum Rezipientenstamm (WT) dargestellt. Beide Sequenzen können durch die T- nach A-Substitution im Kodon 167 |

3

des $\beta$-Tubulin-Gens unterschieden werden.

Fig. 4:            Vergleich der Gen-Organisation von $\beta$-Tubulin-Genen verschiedener Pilze

Fig. 5:            Konstruktion des Vektors pAUL1. Bei der Konstruktion des Vektormoleküls wurden zwei Ausgangsplasmide verwendet. Bei dem bakteriellen Plasmid pACYC184 wurde das Chloramphenikol-Resistenz-Gen genutzt, um eine Selektion in Bakterien zu ermöglichen. Vom Vektor pMW1 stammt schließlich das Hygromycin-Gen, welches mit dem Promoter des pcbC-Gens fusioniert ist.

Abkürzungen: ori, bakterieller Replikations-Ursprung; A, AatII; B, BamHI; E, EcoRI; Hc, HincII; Hd, HindIII; N, NcoI; S, SalI

Zum Offenbarungsgehalt der vorliegenden Anmeldung soll auch der Inhalb der deutschen prioritätsbegründenden Anmeldung mit der Nummer P 43 04 312.7 zählen.

Beispiele

1. Isolierung des $\beta$-Tubulin-Gens von A. chrysogenum

Konstruktion einer Lambda-Genbank aus genomischer DNA von Acremonium chrysogenum:

Zur Isolierung des $\beta$-Tubulin-Gens wurde die in Kück et al. (1989) Appl. Microbiol. Biotechnol 31: 358-365 beschriebene Genbank des A. chrysogenum-Stammes H780 verwendet. Die Herstellung der Genbank ist detailliert in der Patentanmeldung DE 3912822 A1 aufgeführt. Die Isolierung von DNA aus Lambda-Klonen wurde verändert nach Frischauf et al. (1983) J. Mol. Biol. 170:827-842 vorgenommen.

Die Plasmidisolierung aus E.coli durch alkalische Lyse und anschließende CsCl-Ethidiumbromid-Dichtegradienten-Zentrifugation erfolgte nach Birnboim und Doly (1979) Nucl. Acids Res. 7:1513-1522.

Hybridisierungsexperimente zur Identifizierung rekombinanter Lambda-Phagen mit dem $\beta$-Tubulin-Gen :

Zur Identifizierung solcher rekombinanten Lambda-Phagen, welche das $\beta$-Tubulin-Gen tragen, wurde als Hybridisierungssonde das rekombinante Plasmid p$\beta$5 verwendet. Dieses Plasmid trägt das $\beta$-Tubulin-Gen von Aspergillus nidulans. Die Überprüfung von $5 \times 10^3$ rekombinanten Phagen lieferte zwei unabhängige und strukturell verschiedene Lambda-Klone, die eindeutige Homologien zu dem Plasmid p$\beta$5 aufwiesen. Die beiden Lambda-Klone erhielten die Bezeichnung L-T6 und L-C5.

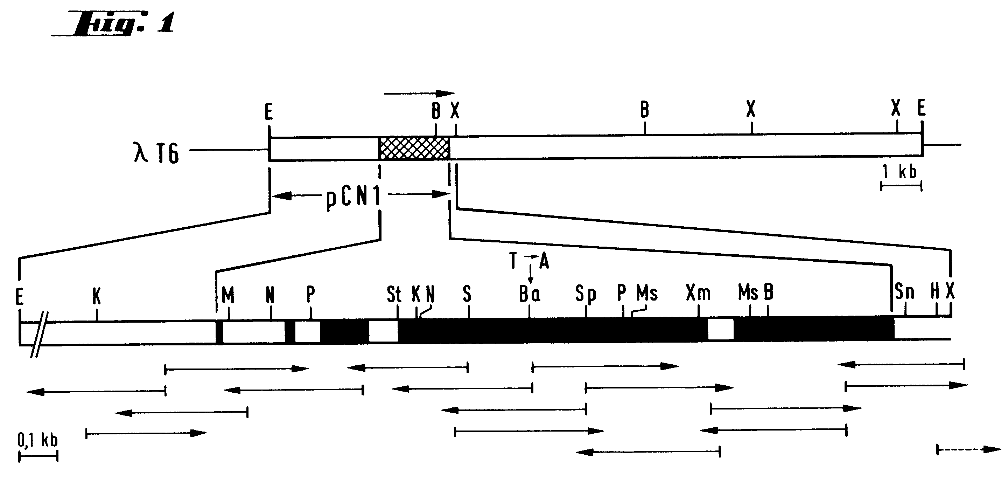

Im folgenden haben wir die weiteren Arbeiten mit dem Klon L-T6 durchgeführt, dessen Restriktionskarte in der Abbildung 1 wiedergegeben ist. Durch den Einsatz verschiedener Restriktionsenzyme konnte eine orientierende Restriktionskarte für die Enzyme XbaI, EcoRI und BamHI erstellt werden (Abb. 1).

Die Hybridisierungsergebnisse zeigten, daß das $\beta$-Tubulin-Gen auf einem 4,7 kb großen EcoRI-XbaI-Fragment des Klons L-T6 lokalisiert ist. Dieses 4,7 kb Restriktionsfragment wurde anschließend in den EcoRI-XbaI-restringierten Vektor pBluescript-IIKS + (Stratagene, Heidelberg) kloniert. Das entsprechende rekombinante Plasmid hat eine Größe von 7,7 kb und erhält die Bezeichnung pCN1 (siehe auch Abb. 1).

Methode: Southern Blots und DNA/DNA-Hybridisierungen. Die in Agarosegelen elektrophoretisch aufgetrennte DNA wurde nach Denaturierung und Neutralisierung auf Nitrozellulosefilter bzw. Hybond N Nylon Membran übertragen (Southern (1975) J. Mol. Bio. 98:503-517). Koloniefilterhybridisierungen wurden nach Grundstein und Hogness (1975) Proc. Natr. Acad. Sci USA 72:3961-3965 durchgeführt. Anschließend wurde die DNA durch UV-Bestrahlung im Stratalinker (Stratagene) an die Membran fixiert. Nach einer Vorhybridisierung der Filter für 1 bis 2 h bei 37°C in 5x SSPE, 0,2 % SDS, 100 $\mu$g/ml Heringssperma-DNA und 50 % Formamid, erfolgte die Hybridisierung nach Zugabe der mittels Oligolabeling mit (alpha $^{32}$P) ATP nach Feinberg und Vogelstein (1983) Anal. Biochem. 132:6-13 radioaktiv markierten DNA-Sonde zum Vorhybridisierungspuffer. Nach Inkubation für 16 h bei 37°C wurde die unspezifisch gebundene DNA durch Waschen der Filter in 5x SSPE, 0,2 % SDS entfernt. Anschließend wurden die Filter für 5 h bis 7 d mit Röntgenfilmen Fuji X-Ray RX und mit Verstärkerfolie Kodak X-Omatic Regulär bei -20°C exponiert.

Methode: E.coli-Klonierung und Konstruktion des Plasmides pCN1. Das 4,7 kb große EcoRI-XbaI-Fragment des Klons L-T6 wurde durch präparative Elution aus 1 %igen Agarosegelen erhalten (nach Silhavy et al. (1984) Experiments with gene fusion. Cold Spring Harbour Laboratory Press, New York pp 164-165). In einem Volumen von 40 $\mu$l 1x Ligationspuffer wurden 5 $\mu$g DNA des L-T6 Fragments und 1 $\mu$g XbaI-EcoRI-verdaute DNA von pBluescript-IIKS + (Stratagene, Heidelberg) mit 5 Enzymeinheiten T4-DNA-Ligase (Boehringer, Mannheim) über Nacht bei 14°C ligiert. Anschließend wurde 1/20 Volumen des Ligationsansatzes für die Transformation in den E.coli-Stamm XL1 blue (Bullock et al. (1987) Biotechniques 5: 376-379) eingesetzt. Die Herstellung kompetenter Zellen sowie die Durchführung der Elektroporation erfolgten unter Verwendung eines Biorad Elektroporationsgerates (Gene PulserTM, Capacitance Extender, Pulse Controler) nach Vorschrift des Herstellers.

Anzucht des E.coli Stammes XL1 blue (Bullock et al. 1987) in Schüttelkulturen bei 37°C mit 270 Upm in LB-Medium (1 % Bacto-Trypton, 0,5 % Hefeextrakt, 0,5 % NaCl, pH 7,2) mit 5 $\mu$g/ml Tetrazyklin. Selektionsmedium enthält zusätzlich 80 $\mu$g/ml Ampicillin.

2. DNA-Sequenzierung des $\beta$-Tubulin-Gens aus A. chrysogenum

Um die Identität des $\beta$-Tubulin-Gens exakt zu ermitteln, wurde eine Total-Sequenzierung des $\beta$-Tubulin-Gens von A. chrysogenum durchgeführt. Die Sequenzierung der Doppelstrang-DNA wurde ausschließlich mit individuell synthetisierten Primern (= Oligonukleotide) vorgenommen. Die Anfangssequenzen wurden durch Verwendung der sogenannten M13-Sequenzierungsprimer ermittelt. Diese Oligonukleotide besitzen eine Homologie zum $\beta$-Galaktosidase-Gen des Klonierungsvektors. Die entsprechende Strategie dieser DNA-Sequenzierung und die daraus resultierende Fein-Restriktionskarte ist in der Tab. 1 (unten) ersichtlich. Die Ergebnisse der Sequenzierung (2206 bp) sind in Tab. 2 Teil A und B dargestellt. Wiedergegeben ist die Sequenz des Einzelstrangs und davon abgeleitet die Übersetzung in die entsprechende Aminosäuresequenz. Deutlich wird, daß bei A. chrysogenum, ähnlich wie bei anderen Hyphenpilzen, die Aminosäure-kodierende Sequenz (Exonen) durch mehrere Intronen unterbrochen wird. Aus dieser Analyse kann geschlossen werden, daß das $\beta$-Tubulin-Gen von A. chrysogenum für 447 Aminosäuren kodiert. Bei den Intron-Sequenzen fällt auf, daß bei allen Intronen sogenannte typische Konsensus-Sequenzen am 5'- und am 3'-Ende identifiziert werden können (Tab. 3).

Die Identität des Gens wurde außerdem durch einen Vergleich der Aminosäuresequenzen von $\beta$-Tubulin-Genen verschiedener Organismen deutlich. In der Tab. 4 ist ein Vergleich der Aminosäuresequenz von A. chrysogenum mit sechs verschiedenen Sequenzen dargestellt. Die Sequenzähnlichkeiten lassen den eindeutigen Schluß zu, daß wir das $\beta$-Tubulin-Gen von A. chrysogenum isoliert haben.

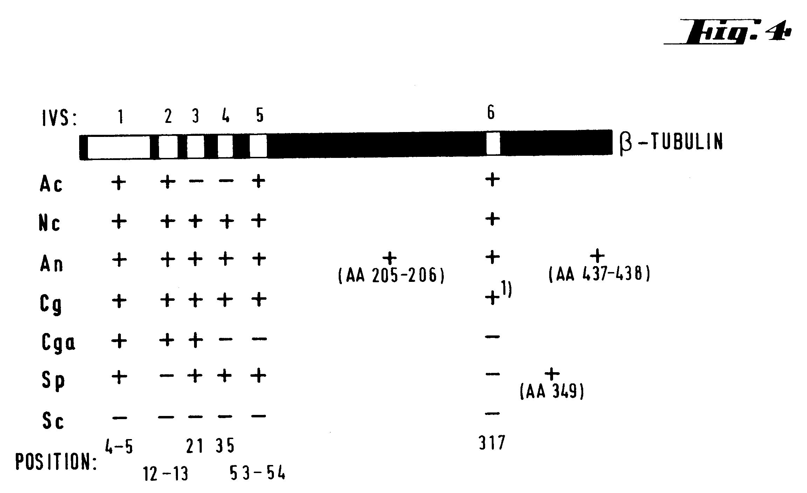

Schließlich haben wir einen Vergleich der Intronen des $\beta$-Tubulin-Gens mit den sog. Konsensus-Sequenzen von Intronen anderer Pilze vorgenommen (Tab. 3). Danach können am 5'-Ende der Acremonium-Intronen typische Konsensus-Sequenzen nachgewiesen werden, wie sie auch bei anderen Eukaryonten zu finden sind. Außerdem interessant ist die Verteilung der vier A. chrysogenum-Intronen im $\beta$-Tubulin-Gen (Fig. 4). Die Position dieser Intronen ist im Vergleich zu anderen Pilzen sehr konservativ und wird an identischer Stelle z. B. auch bei Neurospora crassa (N.c.) oder auch bei Aspergillus nidulans (A.n.) gefunden. Eine ähnliche Intronverteilung findet sich auch bei der Spalthefe Schizosaccharomyces pombe (S.p.). Lediglich bei der Bäckerhefe Saccharomyces cerevisiae (S.c.) kommen keine Intronen im $\beta$-Tubulin-Gen vor.

Methode: DNA-Sequenzierung. Sequenzierungen wurden mit dem T7-Polymerase$^{TM}$-Sequenzierungskit bzw. mit den Deaza G/A T7 Sequenzierungs$^{TM}$ Mixen (Pharmacia, Freiburg) vorgenommen. Die elelektrophoretische Auftrennung der Proben erfolgte mit 6%-Acrylamid-Harnstoff-Ionengradienten-Gelen nach Biggin et al. (1983) Proc Natl. Acad. Sci. USA 80:3963-3965.

3. In vitro-Mutagenese des $\beta$-Tubulin-Gens

Der Austausch eines Nukleotides in der Wildtypsequenz erfolgte durch die Konstruktion eines Oligonukleotidpaares, welches die Basensubstitution in einem Oligonukleotid enthält und anschließend zur Amplifikation der Wildtypsequenz mittels PCR (polymerase chain reaction) eingesetzt wurde. Das erhaltene Fragment wurde dann durch Restriktion und nachfolgende Ligation in das Wildtyp-Gen einkloniert.

Das vollständige, in vitro mutierte $\beta$-Tubulin-Gen konnte durch die anschließende Ligation der folgenden drei Restriktionsfragmente erhalten werden. Das resultierende Plasmid erhält die Bezeichnung "pCN3":

a) das 155 bp große BalI-SphI-Fragment, welches die Basensubstitution enthält und durch PCR-Amplifikation entstanden ist.

Mit dem Oligonukleotidpaar 375 x 376 wurde ein 155 bp großes Fragment des $\beta$-Tubulin-Gens amplifiziert. In diesem Fragment ist das Kodon für Aminosäure 167 gelegen. Bei der Synthese des Oligonukleotids 375 wurde eine Basensubstitution im Vergleich zur Wildtypsequenz eingefügt. Durch diese Substitution wird beim Amplifikationsvorgang eine Mutagenese des Kodons 167 vorgenommen (Tab. 5). Das 155 bp große Fragment enthält an seinen Enden jeweils eine BalI- bzw. eine SphI-Restriktionsschnittstelle, die eine nachfolgende in vitro-Rekombination erlauben.

b) ein 5,0 kb großes SphI-BalI-Restriktionsfragment mit dem 3'-Ende des Wildtyp $\beta$-Tubulin-Gens und allen prokaryontischen Sequenzen des E.coli Vektors pBluescript II KS + (Stratagene, Heidelberg).

c) ein 2,0 kb großes BalI-Restriktionsfragment, welches den 5'-Bereich des Wildtyp $\beta$-Tubulin-Gens enthält.

Durch die in vitro-Rekombination entstand das rekombinante Plasmid pCN3. Dieses Plasmid hat eine Größe von 7,16 kb, bei dem in die EcoRI-XbaI-Restriktionsschnittstelle des Vektors pBlueskiptKSII + das mutierte β-Tubulin-Gen (auf einem 4,2 kb großen EcoRI-XbaI-Fragment) inseriert ist.

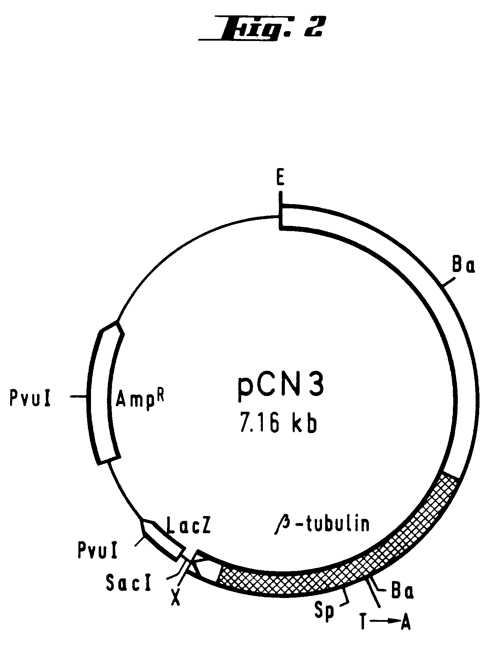

Die Restriktionskarte des Plasmids pCN3 ist in der Fig. 2 wiedergegeben. Repräsentative Schnitte für Restriktionsendonukleasen sind entsprechend gekennzeichnet worden.

Zur Überprüfung, daß im Verlauf der in vitro-Rekombination keine Sequenzveränderungen eingetreten sind, wurde die Komplett-Sequenzierung des mutierten β-Tubulin-Gens vorgenommen. Die Überprüfung der Sequenz wurde mit den Oligonukleotid-Primern, die in der Tabelle 1 aufgeführt sind, durchgeführt. Die Sequenz ergibt sich aus den Daten der Tab. 2.

Methode: In vitro-Mutagenese. Zu 10 ng Plasmid-DNA werden jeweils 0,3 $\mu$g Oligonukleotid-DNA, 1x Replithermpuffer (Biozym, Hameln), 0,33 mM dNTPs sowie 2 Enzymeinheiten Replitherm (Biozym, Hameln) hinzugegeben. Die Amplifikation erfolgte in 40 Zyklen mit jeweils 1 min 92°C, 2 min 60°C, 0,5 min 72°C (verändert nach Saiki et al. (1985) Science 230:1350-1354).

## 4. Transformation von A. chrysogenum mit dem mutierten β-Tubulin-Gen

Bei der Transformation des A. chrysogenum Stammes A3/2 wurde die Regenerationsrate der Protoplasten auf CCM-Medien mit unterschiedlicher Saccharose-Konzentration ermittelt. Dabei zeigte sich eine deutliche Zunahme der Regenrationsfähigkeit bei Verwendung von Medien mit hohen Saccharose-Konzentrationen. Bei Verwendung von 20 % Saccharose liegt die Regenerationsrate bei über 1 %. Im Vergleich zu dem bisher verwendeten CCMS-Medium mit 10.8 % liegt die Regenrationsfähigkeit somit etwa um den Faktor 10 höher. Da die Regenerationsfähigkeit der Protoplasten die wesentliche Voraussetzung für die Transformationrate ist, konnte durch Entwicklung des genannten Medium die effiziente Transformation des A. chrysogenum Stammes A3/2 erfolgen.

Wir haben Acremonium-Transformanten sowohl mit dem gereinigten mutierten β-Tubulin-Gen transformiert als auch das rekombinante Plasmid pCN3 eingesetzt.

Im Verlauf der Transformation werden die Protoplasten zunächst auf nicht-selektiven Medien ausplattiert, nach eintägiger Regeneration der Protoplasten wird eine Überschichtung der Platten mit Selektiv-Agar vorgenommen (15 $\mu$g Benomyl/ml Überschichtungsmedium). Die putativen Transformanten können in der Regel nach 5 bis 8 Tagen optisch erkannt werden. Bei einem anschließenden Transfer wurden die Pilzkolonien erneut auf Selektivmedien überführt und nach entsprechender Regeneration für molekulare Analysen verwendet.

Der Nachweis einer erfolgreichen DNA-Transformation wurde durch physiologische Tests (Wuchstest auf Selektivmedien) und molekulare Analysen (Southern-Hybridisierung) durchgeführt.

Die Benomyl-resistenten Transformanten zeigen gegenüber dem Ausgangsstamm ein deutlich verschiedenes Wuchsverhalten. Bei allen Transformanten ist gegenüber dem Ausgangsstamm ein deutliches Wachstum vorhanden. Bereits bei einer Konzentration von 3 $\mu$g/ml Benomyl im Medium wächst der Ausgangsstamm nur sehr reduziert.

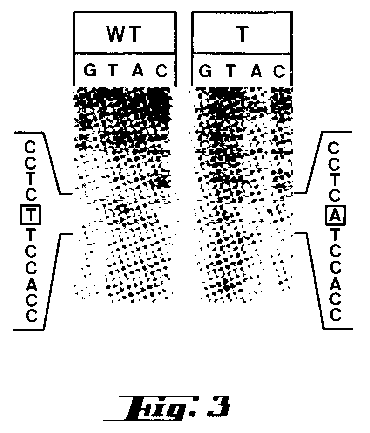

Außerdem wurden die Transformanten durch eine Sequenzanalyse molekular charakterisiert. Zu diesem Zweck wurde ein Fragment des β-Tubulin-Gens durch die Technik der PCR-Amplifikation synthetisiert. Hierbei wurde die DNA von Transformanten von Acremonium chrysogenum gewonnen und anschließend für die PCR-Amplifikation mit den Oligonukleotid-Startermolekülen Nr. 341 und Nr. 376 (siehe Tab. 1) amplifiziert. Schließlich erfolge die direkte DNA-Sequenzierung mit Hilfe der sogenannten Kettenabbruch-Reaktion. Als Referenz wurde parallel die DNA des Rezipientenstammes von Acremonium chrysogenum sequenziert. Die Fig. 3 gibt ein repräsentatives Ergebnis wieder; in der Teilsequenz ist die Basen-Substitution innerhalb des β-Tubulin-Gens bei einem Transformanten (T) im Vergleich zum Rezipientenstamm (WT) wiedergegeben.

Methode. Direkte DNA-Sequenzierung von PCR-amplifizierten DNA-Fragmenten:

Die Gesamt-DNA von Pilzstämmen (Rezipientenstamm, Benomyl-resistente Transformanten) wurde - wie bereits beschrieben - gewonnen. Im Anschluß daran erfolgte die Amplifikation eines Teilfragmentes des β-Tubulin-Gens mit Hilfe der Oligonukleotide Nr. 341 und Nr. 376 (Saiki et al. (1985), Science 230: 1350-1354). Anschließend wurde das DNA Fragment mit Ethanol präzipitiert und nach Aufnahme in Pufferlösung für die direkte Sequenzierung nach der Kettenabbruch-Methode (Sanger et al. (1977), Proc Natl Acad Sci US 74: 5463-5467) verwendet. Danach wurden die Sequenzreaktionen auf denaturierenden Polyacrylamidgelen aufgetrennt und für die folgende Autoradiographie verwendet.

Methode: Transformation von A. chrysogenum.:

Verändert nach Skatrud et al. (1987) Curr. Genet. 12:337-348. Anzucht von A. chrysogenum (ATCC 14553) bei 27°C. Die Flüssigkulturen wurden mit 200 Upm in CCM-Medium (0,3 % Saccharose, 0,05 % NaCl, 0,05 % K2HPO4, 0,05 % MgSO4 • 7H2O, 0,001 % FeSO4 • 7H2O, 0,5 % Tryptic-Soy-Broth, 0,1 % Hefeextrakt, 0,1 % Fleischextrakt, 0,15 % Dextrin, pH 7.0) geschüttelt. Zwei 500 ml Erlenmeyerkolben mit je 100 ml CCM werden mit 4 bis 6 d alten Myzel angeimpft. Nach 36 bis 48 h Anzucht wird das Myzel abfiltriert und mit 0.8 M KCl gewaschen. Ca. 3 g Myzel werden in 20 ml Protoplastenpuffer (PP) mit 50 mM DTT 15 min bei 27°C inkubiert. Nach Zentrifugation 10 min mit 15000 Upm (SS34) wird das Pellet in 20 ml PP mit 10 mg Novozym/ml resuspendiert und 45 bis 60 min bei 27°C mit 100 Upm inkubiert. Die Protoplasten werden über eine Glasfritte (Porengröße 2, (Schott)) vom Myzel getrennt, 3 min mit 2500 Upm pelletiert, mit 10 ml PP gewaschen, erneut abzentrifugiert und auf einen Protoplastentiter von 1 x 10⁹/ml Transformationspuffer (TP) eingestellt. 20 $\mu$g ($\approx$ 10 bis 20 $\mu$l) Plasmid-DNA werden mit 1 x 10⁸ Protoplasten vermischt und 10 min auf Eis inkubiert. 200 $\mu$l PEG (25 % PEG in TP) werden zugegeben, der Ansatz wird gemischt und 20 min bei Raumtemperatur inkubiert. Die Hälfte des Ansatzes wird zu 5 ml Topagar (CCMS mit 0,8 % Agar, ca. 50°C) gegeben und nach Vermischen auf einer CCMS-Platte ausplattiert. Verdünnungen einer 0-Kontrolle mit $H_2O$ bzw. PP dienen zur Bestimmung der Sporenanzahl und der Regenerationsrate. Nach 24 bis 48 h Bebrütung werden die Platten mit 5 ml Selektionsagar (0.8 M NaCl, 0,8 % Agar, 15 $\mu$g Benomyl/ml) überschichtet. Transformanten erscheinen nach 5 bis 12 d.

Die Anzucht der Transformanten wurde auf Selektivmedien (CCMS + 10 $\mu$g Benomyl/ml) vorgenommen.

Die Southern-Hybridisierungen der Transformanten-DNA erfolgte mit radioaktiv markierten Sonden wie unter 1) beschrieben.

5. Konstruktion eines Transformationsvektors pAUL1, der kein $\beta$-Lactamase-Gen enthält

Bei der Transformation von A. chrysogenum sind solche Vektoren interessant, die kein $\beta$-Lactamase-Gen enthalten. Dies ist insbesondere wichtig, wenn durch Transformation die Cephalosporin-Biosynthese beeinflußt werden soll. Zwar kann man nicht unbedingt von einer Expression des bakteriellen $\beta$-Lactamase-Gens ausgehen, jedoch ist denkbar, daß auch geringste Expressionsraten dieses prokaryotischen Gens in dem Eukaryonten A. chrysogenum zum Cephalosporin-Abbau führen können.

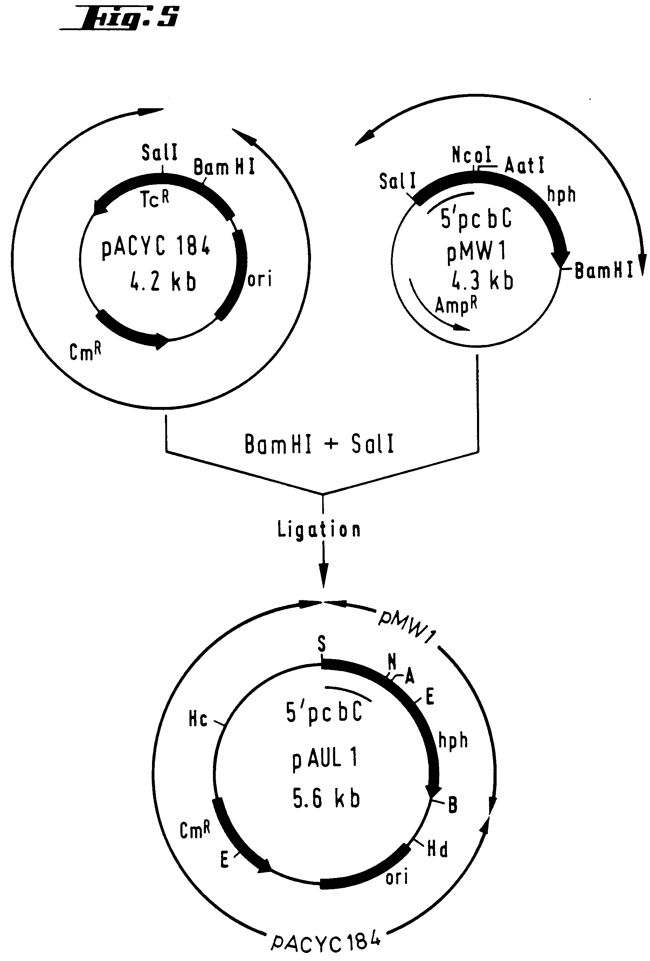

Wir haben, ausgehend von dem bereits bekannten Vektor pMW1, die Konstruktion eines $\beta$-Lactamase-Gen-freien Vektors durchgeführt. Zu diesem Zweck wurde der Vektor pAUL1 konstruiert, der als Selektionsgen in E.coli ein Chloramphenikol-Gen enthält (Fig. 5). Wie in der Restriktionskarte in der Fig. 5 zu erkennen ist, wurde der Vektor pAUL1 auf der Basis des bakteriellen Plasmids pACYC184 erstellt. Der Vektor pAUL1 enthält außerdem das zur Selektion in A. chrysogenum notwendige Hygromycin-Resistenzgen, das unter der Kontrolle des pcbC-Promotors steht. Mit dem Konstrukt pAUL1 wurden A. chrysogenum-Protoplasten transformiert und anschließend auf Hygromycin-Medium selektioniert.

Tatsächlich konnten wir, ähnlich wie mit dem Vektor pMW1, A. chrysogenum-Transformanten mit der unter 5. beschriebenen Methode isolieren. Allerdings ist die Transformationsfrequenz um den Faktor 10 geringer, verglichen mit dem Vektor pMW1. Durch das Verfahren der Southern-Hybridisierung konnte auf Autoradiographien gezeigt werden, daß in allen Acremonium-Transformanten Vektor-DNA enthalten ist.

Ähnlich wie für pMW1 bereits gezeigt, entsteht ein komplexes Hybridisierungsmuster. Dieses Ergebnis läßt darauf schließen, daß nicht nur eine Amplifikation, sondern auch Rekombination des Vektors in den Transformanten erfolgt ist. Deutlich ist bei nahezu allen Transformanten ein 3,5 kb großes Fragment im Autoradiogramm zu erkennen. Dieses Fragment enthält die endogene pcbC-Genkopie, die erwartungsgemäß bei Verwendung des Plasmids pMW1 als Hybridisierungssonde nachweisbar ist.

Methode: Plasmid-Konstruktion und Acremonium-Transformation.

Zur Konstruktion des Vektors pAUL1 wurden die beiden folgenden Ausgangsplasmide verwendet: 1. pACYC184 (Chang und Cohen (1976) J. Bacteriol. 143:1141-1156),

2. pMW1 (Kück et al. 1989).

5 $\mu$g des Plasmids aus 1. wurden mit den Enzymen SalI und BamHI hydrolysiert. 10 $\mu$g des Plasmids aus 2. wurden mit SalI und BamHI hydrolysiert. Durch diese Restriktion entsteht ein 1,5 kb großes BamHI-SalI-Restriktionsfragment, welches das hph-Gen mit dem 5'pcbC-Promoter enthält. Das Fragment wurde gelelektrophoretisch gereinigt und präparativ isoliert. Anschließend erfolgte die Ligation des Fragmentes mit dem BamHI- + SalI-restringierten Vektor pACYC184. Die anschließende E.coli-Transformation und Koloniefilter-Hybridisierung zur Identifizierung rekombinanter Plasmide wird nach dem Verfahren - wie unter 1. beschrieben - durchgeführt. Der Vektor pAUL1 wird in Fig. 5 dargestellt.

6. Kotransformation von A. chrysogenum unter Verwendung des mutierten $\beta$-Tubulin-Gens

Bei der Kotransformation von A. chrysogenum wurde der Vektor pCN3 zusammen mit den Vektoren pMW1 bzw. pAUL1 in A. chrysogenum transformiert. Die anschließende Analyse auf Selektivmedien, die entweder Benomyl bzw. Hygromycin enthielten, und die folgende Southern-Hybridisierung machten deutlich, daß 70 bis 80 % der Transformanten beide DNA-Moleküle aufgenommen haben. Dadurch wird belegt, daß der Vektor pCN3 zur Kotransformation geeignet ist. Es wird außerdem dadurch gezeigt, daß beliebige DNA in A. chrysogenum mit Hilfe des Vektors pCN3 transformiert werden kann.

Methode der Kotransformation: Es wurde zur DNA-Transformation die unter 4. beschriebene Transformationsmethode von A. chrysogenum verwendet. Dabei wurde DNA des Plasmids pCN3 im Gewichtsverhaltnis 1:1 mit den Plasmiden pMW1 bzw. pAUL1 gemischt. Anschließend erfolgte die Selektion auf Benomyl-haltigen Selektivmedien. Die physiologischen Teste wurden anschließend fortgeführt auf Hygromycin-haltigen Medien, denen Hygromycin B zugesetzt worden war. Die molekulare Analyse wurde durch die Southern-Hybridisierung mit radioaktiv-markierten Vektor-Sonden durchgeführt. Das Verfahren - wie unter 4. beschrieben - wurde verwandt.

**Tab.1**     Sequenz der verwendeten Oligonukleotide für die DNA-Sequenzierung und in vitro-Mutagenese.

| Bezeichnung | Sequenz | | |
|---|---|---|---|
| Oligo 340 | 5′ CCGTTGAAGTAGACGCTCATGC | 3′ | |
| Oligo 341 | 5′ GCCAAGGGCCACTACACTGAGGGT | 3′ | |
| Oligo 359 | 5′ CAGGAATGTTGTTGCTCGACGC | 3′ | |
| Oligo 360 | 5′ TACGACATCTGCATGCGTACC | 3′ | |
| Oligo 373 | 5′ TGACCTGCTCTGCCATCTTG | 3′ | |
| Oligo 374 | 5′ GGAGTAGGTGGCCATCATGC | 3′ | |
| Oligo 375 | 5′ GATGGCCACCTACTCCGTCG | 3′ | |
| Oligo 376 | 5′ GGGTACGCATGCAGATGTCG | 3′ | |
| Oligo 377 | 5′ TGAGGGCATGGACGAGATGG | 3′ | |
| Oligo 378 | 5′ TGATGGAGAAGAGTGTGTG | 3′ | |
| Oligo 433 | 5′ CCACCACACACTCTTCTCC | 3′ | |
| Oligo 434 | 5′ CCATCTCGTCCATGCCCTCA | 3′ | |
| Oligo 439 | 5′ ATCATCAGCCAGATTGG | 3′ | |
| Oligo 440 | 5′ GGAACAAGACAATAGAGC | 3′ | |
| Oligo 441 | 5′ CGTTCTATTTATTTCAATTCG | 3′ | |
| univ. primer | 5′ GTAAAACGACGGCCAGT | 3′ | Pharmacia |

Tab. 2 Teil A

```
   1 CGCAATTTGT CCCACATCAT CAGCCCATTG GTCATCACCC CCCCTCAGGTA CCAACCAGTGA ACACCTTACTG TAAAGGCTCC   83

  84 CGCTGTCAGG CCCAGCAGCA GCTCCAGCAC TCACCGCAAG AGGCCGGGGTC CCGTGGCGGTG AGGACGGGACC AATTTCCATC   166

 167 CAGCACCTCA CCCACCTCTC CCCCAGCAAA AATCCAACCA TCCACCACACA CTCTTCTCCAT CACTCACACCC GACCACCCCC   249

 250 CATCTCCACG GAACTCAAGC TCTGTTGCTC AGCACAGCTC TCGAGGCCCTC GCTCTAGCTAC CGATCACGTCC TTTTCCCTTC   332

 333 AATAATCCTC AAA ATG CGT GAG ATT gtgagtcctc caacggacgc ccaacgcgtc cctcgcggtgc ccctgatttac c   410
             MET Arg Glu Ile
               1

 411 ccgccgacgg gcgtcgagca acaacattcc tgcccgacac actacccaca gttcgagatg gatcaagaac gtgctgacca tgg   493

 494 acctttttttt tgttcttgtg atatag GTT CAC CTC CAG ACC GGC CAG TGC gtaagttact tctttccgga cacg   567
                           Val His Leu Gln Thr Gly Gln Cys
                                              10

 568 tttgcggtgt gggaggacac agctgatgga cgtgatggaa tag GGC AAC CAG ATT GGT GCT GCT TTC TGG CAG   640
                                                 Gly Asn Gln Ile Gly Ala Ala Phe Trp Gln
                                                                             20

 641 ACC ATC TCT GGC GAG CAT GGC CTC GAC AGC AAC GGT GTC TAC AAC GGC AGC TCT GAG CTC CAA   703
     Thr Ile Ser Gly Glu His Gly Leu Asp Ser Asn Gly Val Tyr Asn Gly Ser Ser Glu Leu Gln
                         30                                      40

 704 CTC GAG CGC ATG AGC GTC TAC TTC AAC GAG gtacgtggatgaactagctacaactgtactgctcgagcagtca   776
     Leu Glu Arg MET Ser Val Tyr Phe Asn Glu
                     50

 777 atgctagtgt gggtcctaac aagctgtgca g GCC TCT GGC AAC AAG TAT GTC CCT CGC GCC GTC CTC GTC   846
                                      Ala Ser Gly Asn Lys Tyr Val Pro Arg Ala Val Leu Val
                                                                   60

 847 GAT CTT GAG CCC GGT ACC ATG GAC GCT GTT CGT GCG GGT CCT TTC GGC CAG CTC TTC CGC CCC   909
     Asp Leu Glu Pro Gly Thr MET Asp Ala Val Arg Ala Gly Pro Phe Gly Gln Leu Phe Arg Pro
                     70                                  80

 910 GAC AAC TTC GTC TTC GGC CAG TCC GGT GCT GGC AAC AAC TGG GCC AAG GGC CAC TAC ACT GAG   972
     Asp Asn Phe Val Phe Gly Gln Ser Gly Ala Gly Asn Asn Trp Ala Lys Gly His Tyr Thr Glu
                 90                                 100

 973 GGT GCC GAG CTC GTC GAC AAC GTC CTC GAT GTC GTC CGC CGC GAG GCC GAG GGC TGC GAC TGC   1035
     Gly Ala Glu Leu Val Asp Asn Val Leu Asp Val Val Arg Arg Glu Ala Glu Gly Cys Asp Cys
         110                                 120

1036 CTC CAG GGC TTC CAG ATC ACC CAC TCC CTG GGT GGT GGC ACT GGT GCC GGT ATG GGC ACC CTG   1098
     Leu Gln Gly Phe Gln Ile Thr His Ser Leu Gly Gly Gly Thr Gly Ala Gly MET Gly Thr Leu
     130                                 140                                 150

1099 CTC ATC TCC AAG ATC CGC GAG GAG TTC CCC GAC CGC ATG ATG GCC ACC TTC TCC GTC GTC CCC   1161
     Leu Ile Ser Lys Ile Arg Glu Glu Phe Pro Asp Arg MET MET Ala Thr Phe Ser Val Val Pro
                                 160                                 170

1162 TCC CCC AAG GTC TCC GAT ACC GTC GTC GAG CCC TAC AAC GCC ACC CTC TCC GTG CAC CAG CTC   1224
     Ser Pro Lys Val Ser Asp Thr Val Val Glu Pro Tyr Asn Ala Thr Leu Ser Val His Gln Leu
                             180                                 190

1225 GTT GAG CAC TCC GAC GAG ACC TTC TGT ATC GAC AAC GAG GCC CTC TAC GAC ATC TGC ATG CGT   1287
     Val Glu His Ser Asp Glu Thr Phe Cys Ile Asp Asn Glu Ala Leu Tyr Asp Ile Cys MET Arg
                         200                                 210

1288 ACC CTC AAG CTG TCT AAC CCC TCC TAC GGC GAC CTG AAC TAC CTC GTC TCC GCT GTC ATG TCT   1350
     Thr Leu Lys Leu Ser Asn Pro Ser Tyr Gly Asp Leu Asn Tyr Leu Val Ser Ala Val MET Ser
                         220                                 230

1351 GGT GTC ACC ACC TGC CTC CGC TTC CCC GGT CAG CTG AAC TCT GAC CTG CGC AAG CTG GCT GTC   1413
     Gly Val Thr Thr Cys Leu Arg Phe Pro Gly Gln Leu Asn Ser Asp Leu Arg Lys Leu Ala Val
                     240                                 250

1414 AAC ATG GTT CCC TTC CCT CGT CTG CAC TTC TTC ATG GTC GGC TTC GCC CCC CTG ACC AGC CGT   1476
     Asn MET Val Pro Phe Pro Arg Leu His Phe Phe MET Val Gly Phe Ala Pro Leu Thr Ser Arg
                     260                                 270

1477 GGT GCC CAC TCC TTC CGC GCC GTC AGC GTC CCC GAG CTC ACC CAG CAG ATG TTC GAC CCC AAG   1539
     Gly Ala His Ser Phe Arg Ala Val Ser Val Pro Glu Leu Thr Gln Gln MET Phe Asp Pro Lys
                     280                                 290

1540 AAC ATG ATG GCT GCC TCC GAC TTC CGC AAC GGC CGC TAC CTG ACC TGC TCT GCC ATC TT gta   1601
     Asn MET MET Ala Ala Ser Asp Phe Arg Asn Gly Arg Tyr Leu Thr Cys Ser Ala Ile Ph
             300                                 310

1602 agttataaag atacgccgag ctctattgtc ttgttccatc tgaagctaac atggaaacag C CGT GGC AAG GTC GCC   1677
                                                                      e Arg Gly Lys Val Ala
                                                                              320
```

Tab. 2 Teil B

```
1678 ATG AAG GAG GTC GAG GAC CAG ATG CGC AAC GTC CAG AGC AAG AAC TCG TCC TAC TTC GTC GAG    1740
     MET Lys Glu Val Glu Asp Gln MET Arg Asn Val Gln Ser Lys Asn Ser Ser Tyr Phe Val Glu
                                 330                                 340
1741 TGG ATC CCC AAC AAC ATC CAG ACC GCT CTC TGC GCC ATT CCT CCC CGT GGC CTC AAG ATG TCC    1803
     Trp Ile Pro Asn Asn Ile Gln Thr Ala Leu Cys Ala Ile Pro Pro Arg Gly Leu Lys MET Ser
                             350                                 360
1804 TCC ACC TTC ATC GGC AAC TCC ACC TCC ATC CAG GAG CTG TTC AAG CGT GTC GGT GAG CAG TTC    1866
     Ser Thr Phe Ile Gly Asn Ser Thr Ser Ile Gln Glu Leu Phe Lys Arg Val Gly Glu Gln Phe
                         370                                 380
1867 ACT GCC ATG TTC CGT CGC AAG GCT TTC CTG CAT TGG TAC ACT GGT GAG GGC ATG GAC GAG ATG    1929
     Thr Ala MET Phe Arg Arg Lys Ala Phe Leu His Trp Tyr Thr Gly Glu Gly MET Asp Glu MET
                     390                                 400
1930 GAG TTT ACC GAG GCC GAG TCC AAC ATG AAC GAC CTC GTC TCC GAG TAC CAG CAG TAC CAG GAT    1992
     Glu Phe Thr Glu Ala Glu Ser Asn MET Asn Asp Leu Val Ser Glu Tyr Gln Gln Tyr Gln Asp
                 410                                 420
1993 GCT GGC ATC GAC GAG GAG GAG GAG GAA TAC GAG GAG GAG CTC CCC CTC GAG GGT GAG GAA TAA    2055
     Ala Gly Ile Asp Glu Glu Glu Glu Glu Tyr Glu Glu Glu Leu Pro Leu Glu Gly Glu Glu TER
             430                                 440                                 447
2056 AAAAAAAAGC TCGCCACCAG AGGCTTGCCG TATACACGGT CCGGCGCGTC GTTCCCGCCA ACTGTGGTAA CCTTTTGAAG TTT 2138

2139 GCAGCCTGTT GCGTTCTATT TATTTCAATT CGGGGTTGTG GAGGTAATTG TGAGAATGGG GTTCTAGA                    2206
```

```
IVS1:   5' - GTGAGT ...: GCTGACC ...  27   ... TAG - 3'
IVS2:      - GTAAGT ... GCTGATG ...   12   ... TAG -
IVS3:      - GTACGT ... GCTAGTG ...   19   ... CAG -
IVS4:      - GTAAGT ... GCTAACA ...    6   ... CAG -
```

Konsensus-Sequenz von

```
N.c.:      - GTACGT ... GCTGACT ...   7-18  ... CAG -
              A          A   A   A
A.n.:      - TGTAAGT... CTAAC        .........  TTACAGC -
             G   G          G                   CCC
S.c.:      - GTATGT ... TACTAAC ...   18-53 ... TAG -
                                                 C
h.e.:      - GTAAGT .......................... CAG -
```

Tab. 4

```
  1 MREIVIILQTGQCGNQIGAAFWQTISGEHGLDSNGVYNGSS-ELQLERMSVYFNEASGNKYVPRAVLVDLEPGTMDAVRAGPFGQLFRPDNFJFGQSGAGNN
  1 M.............................AS.....T.-.......N...................................................
  1 M..............GS....T.-D......N...................C.....5...........................................
  1 M...I.ISA..Y.........E..C......F..T.H.T.-DI.K..LN.......SG.W...SIN.....W.I....MSAI.N.......YI....S...V
  1 M.....I.G.........K..EVV.D...I.PT.T.H.D.-D.....IN.......T.GR.....I.M......S..S..Y..I...........T.....
  1 M...L.V.G.......GSK..EV.CD...V.PT.R...D.AD.....IN.Y.....GR.......M.........SI.S..Y..I................
  1 M.....I.A.........K..QT..D...I.PT.S.H.D.-D.....IN.Y...T.........I...........S..S.....I................

101 WAKGHYTEGAELVDNVLDVVRREAEGCDCLQGFQITHSLGGGTGAGMGTLLISKIREEFPDRMMATFSVVPSPKVSDTVVEPYNATLSVHQLVEHSDETF
101 ...........Q...................................................................................N.....
101 ............V.......................................................................................
101 ............S.M..I........S.................S......F..K..L..........L....T.........................
101 ............I.S......K...S........VC........S.............Y...L.....................................NA..CM
102 ............I.A......K...N........VC........SQ.............Y.....L...F..............................NA..CM
101 ............S......K.S.S........L.............S.............Y...I.N...M............................NT...Y

201 CIDNEALYDICMRTLKLSNPSYGDLNYLVSAVMSGVTTCLRFPGQLNSDLRKLAVNMVPFPRLHFFHVGFAPLTSRGAHSFRAVSVPELTQQMFDPKNMM
201 ..........................H...........VS.................................................H..........
201 ..........................H...........................W..................Y..........................
201 ..........Q.....NQ.......N...S.......:S..Y..............L..........Y...AI.SQ...SLT.........EA....
201 VL.........F.....TT.TF....H.I......I.C..........A.......LI...............T......SQQY..LT.........W.A....
202 VL.........F......T..F....H.I..T.....CS...............LI...............SQQYISLT.........W.A....
201 ...........F.....TT.T....H....T.......A...............P.........QQY..LT.........S....

301 AASDFRNGRYLTCSAIFRGKVAMKEVEDQMRNVQSKNSSYFVEWIPNNIQTALCAIPPRGLKMSSTFIGNSTSIQELFKRVGEQFTAMFRRKAFLHWYTG
301 ....................S...........N..............V...S..............V....A.......I....................
301 ....................S..........I...Q..........S...S..............................D..................
301 ...A.P.......VA.F.....SV.....E.HK.....D.........V...V.SVA.Q..D.AA...A...............D..S...K.........S
301 C.A.P.H......A..L....RMST...DE..L...N..............VKSSV.D...K.....A........A...M....S...............
302 C.A.P.H......A..M...MMST...DE.IL....N...............VKSSV.D...T.I..A...V........M.R..S...F...........
301 ...C.P.H.....VA.....RMS....DE..L...N............VK..V.D.........A...........A.......IS...............

401 EGMDEMEFTEAESNMNDLVSEYQQYQDAGIDEEEEEYEEELPLEGEE*
401 ...................................V...........A......*
401 .......................................S.S.G....A..EIM....*
401 .....L..S..................E.TVEDD..VD.NGDFGAPQNQDEPITENFE*
401 ...................................SAE..G.FEG..EEAB*
402 ...............................TA...D.YD...EQVYES*
401 ...............................TA..QG.FE..GEED.A*
```

|       |   |                            |
|-------|---|----------------------------|
| 100%  | – | (1) Acremonium chrysogenum |
| 96%   | – | (2) Neurospora crassa      |
| 95%   | – | (3) Aspergillus nidulans   |
| 77%   | – | (4) Saccharomyces cerevisiae |
| 78%   | – | (5) Chlamydomonas reinhardtii |
| 77%   | – | (6) Arabidopsis thaliana   |
| 82%   | – | (7) Gallus gallus          |

Tab. 5

```
                          MET Ala Thr Phe Ser Val

Wildtypsequenz: 5'- G ATG GCC ACC TTC TCC GTC G -3'


   Oligo 375: 5'  G ATG GCC ACC TAC TCC GTC G  3'

                                    Tyr
```

Sequenzprotokoll

Allgemeine Angaben:

Anmelder:   Hoechst AG
            65926 Frankfurt am Main
            Telefon: (069) 305 - 6031
            Telefax: (069) 35 71 75

Bezeichnung der Erfindung:      ß-Tubulin von Acremonium chrysogenum, seine
                                Herstellung und Verwendung

Anzahl der Sequenzen: 18

Computerlesbare Fassung:
Datenträger:      3,5" HD Diskette
Computer:         386 SX
Betriebssystem:   MS-DOS
Software:         Word Perfect 5.1

Angaben zu SEQ ID NO: 1

Sequenzkennzeichen:

Länge:            23 Basen
Art:              Nukleinsäure
Strangform:       Einzelstrang
Topologie:        linear
Ursprüngliche Herkunft:
Organismus:           Acremonium chrysogenum
Unmittelbare Herkunft:   synthetisch

Sequenzbeschreibung:    SEQ ID NO: 1

CTCGTTGAAG TAGACGCTCA TGC

Angaben zu SEQ ID NO: 2

Sequenzkennzeichen:

Länge:            24 Basen
Art:              Nukleinsäure
Strangform:       Einzelstrang
Topologie:        linear
Ursprüngliche Herkunft:
Organismus:              Acremonium chrysogenum
Unmittelbare Herkunft:   synthetisch

Sequenzbeschreibung:    SEQ ID NO: 2

GCCAAGGGCC ACTACACTGA GGGT

Angaben zu SEQ ID NO: 3

Sequenzkennzeichen:

Länge:            22 Basen
Art:              Nukleinsäure
Strangform:       Einzelstrang
Topologie:        linear
Ursprüngliche Herkunft:
Organismus:              Acremonium chrysogenum

Unmittelbare Herkunft:    synthetisch

Sequenzbeschreibung:    SEQ ID NO: 3

CAGGAATGTT GTTGCTCGAC GC

Angaben zu SEQ ID NO: 4

Sequenzkennzeichen:

Länge:                21 Basen
Art:                  Nukleinsäure
Strangform:           Einzelstrang
Topologie:            linear
Ursprüngliche Herkunft:
Organismus:                Acremonium chrysogenum
Unmittelbare Herkunft:    synthetisch

Sequenzbeschreibung:    SEQ ID NO: 4

TACGACATCT GCATGCGTAC C

Angaben zu SEQ ID NO: 5

Sequenzkennzeichen:

Länge:                20 Basen
Art:                  Nukleinsäure
Strangform:           Einzelstrang
Topologie:            linear

Ursprüngliche Herkunft:

Organismus: Acremonium chrysogenum

Unmittelbare Herkunft: synthetisch

Sequenzbeschreibung: SEQ ID NO: 5

TGACCTGCTC TGCCATCTTG

Angaben zu SEQ ID NO: 6

Sequenzkennzeichen:

Länge: 20 Basen

Art: Nukleinsäure

Strangform: Einzelstrang

Topologie: linear

Ursprüngliche Herkunft:

Organismus: Acremonium chrysogenum

Unmittelbare Herkunft: synthetisch

Sequenzbeschreibung: SEQ ID NO: 6

GGAGTAGGTG GCCATCATGC

Angaben zu SEQ ID NO: 7

Sequenzkennzeichen:

Länge: 20 Basen

Art: Nukleinsäure

Strangform:        Einzelstrang

Topologie:        linear

Ursprüngliche Herkunft:

Organismus:            Acremonium chrysogenum

Unmittelbare Herkunft:    synthetisch


Sequenzbeschreibung:    SEQ ID NO: 7


GATGGCCACC TACTCCGTCG


Angaben zu SEQ ID NO: 8


Sequenzkennzeichen:


Länge:            20 Basen

Art:            Nukleinsäure

Strangform:        Einzelstrang

Topologie:        linear

Ursprüngliche Herkunft:

Organismus:            Acremonium chrysogenum

Unmittelbare Herkunft:    synthetisch


Sequenzbeschreibung:    SEQ ID NO: 8


GGGTACGCAT GCAGATGTCG

Angaben zu SEQ ID NO: 9

Sequenzkennzeichen:

Länge: 20 Basen

Art: Nukleinsäure

Strangform: Einzelstrang

Topologie: linear

Ursprüngliche Herkunft:

Organismus: Acremonium chrysogenum

Unmittelbare Herkunft: synthetisch

Sequenzbeschreibung: SEQ ID NO: 9

TGAGGGCATG GACGAGATGG

Angaben zu SEQ ID NO: 10

Sequenzkennzeichen:

Länge: 19 Basen

Art: Nukleinsäure

Strangform: Einzelstrang

Topologie: linear

Ursprüngliche Herkunft:

Organismus: Acremonium chrysogenum

Unmittelbare Herkunft: synthetisch

Sequenzbeschreibung: SEQ ID NO: 10

TGATGGAGAA GAGTGTGTG

Angaben zu SEQ ID NO: 11

Sequenzkennzeichen:

Länge: 19 Basen

Art: Nukleinsäure

Strangform: Einzelstrang

Topologie: linear

Ursprüngliche Herkunft:

Organismus: Acremonium chrysogenum

Unmittelbare Herkunft: synthetisch

Sequenzbeschreibung: SEQ ID NO: 11

CCACCACACA CTCTTCTCC

Angaben zu SEQ ID NO: 12

Sequenzkennzeichen:

Länge: 20 Basen

Art: Nukleinsäure

Strangform: Einzelstrang

Topologie: linear

Ursprüngliche Herkunft:

Organismus: Acremonium chrysogenum

Unmittelbare Herkunft: synthetisch

Sequenzbeschreibung: SEQ ID NO: 12

CCATCTCGTC CATGCCCTCA

Angaben zu SEQ ID NO: 13

Sequenzkennzeichen:

Länge:                 17 Basen

Art:                   Nukleinsäure

Strangform:            Einzelstrang

Topologie:             linear

Ursprüngliche Herkunft:

Organismus:            Acremonium chrysogenum

Unmittelbare Herkunft:   synthetisch

Sequenzbeschreibung:   SEQ ID NO: 13

ATCATCAGCC AGATTGG

Angaben zu SEQ ID NO: 14

Sequenzkennzeichen:

Länge:                 18 Basen

Art:                   Nukleinsäure

Strangform:            Einzelstrang

Topologie:             linear

Ursprüngliche Herkunft:

Organismus:            Acremonium chrysogenum

Unmittelbare Herkunft:   synthetisch

Sequenzbeschreibung:   SEQ ID NO: 14

GGAACAAGAC AATAGAGC

Angaben zu SEQ ID NO: 15

Sequenzkennzeichen:

Länge:                   21 Basen

Art:                     Nukleinsäure

Strangform:              Einzelstrang

Topologie:               linear

Ursprüngliche Herkunft:

Organismus:                    Acremonium chrysogenum

Unmittelbare Herkunft:   synthetisch

Sequenzbeschreibung:     SEQ ID NO: 15

CGTTCTATTT ATTTCAATTC G

Angaben zu SEQ ID NO: 16

Sequenzkennzeichen:

Länge:                   17 Basen

Art:                     Nukleinsäure

Strangform:              Einzelstrang

Topologie:               linear

Ursprüngliche Herkunft:

Organismus:                    Acremonium chrysogenum

Unmittelbare Herkunft:   synthetisch

Sequenzbeschreibung:     SEQ ID NO: 16

GTAAAACGAC GGCCAGT

Angaben zu SEQ ID NO: 17

Sequenzkennzeichen:

| | |
|---|---|
| Länge: | 2206 Basen |
| Art: | Nukleinsäure mit Aminosäure im kodierenden Bereichen |
| Strangform: | Einzelstrang |
| Topologie: | linear |

Ursprüngliche Herkunft:

| | |
|---|---|
| Organismus: | Acremonium chrysogenum |

Unmittelbare Herkunft:

| | |
|---|---|
| Bibliothek: | genomisch |
| Merkmal: | ß-Tubulingen |

Sequenzbeschreibung:    SEQ ID NO: 17

Tab. 2 Teil A

```
  1 CGCAATTTGT CCCACATCAT CAGCCCATTG GTCATCACCC CCCCTCAGGTA CCAACCAGTGA ACACCTTACTG TAAAGGCTCC    83

 84 CGCTGTCAGG CCCAGCAGCA GCTCCAGCAC TCACCGCAAG AGGCCGGGGTC CCGTGGCGGTG AGGACGGGACC AATTTCCATC   166

167 CAGCACCTCA CCCACCTCTC CCCCAGCAAA AATCCAACCA TCCACCACACA CTCTTCTCCAT CACTCACACCC GACCACCCCC   249

250 CATCTCCACG GAACTCAAGC TCTGTTGCTC AGCACAGCTC TCGAGGCCCTC GCTCTAGCTAC CGATCACGTCC TTTTCCCTTC   332

333 AATAATCCTC AAA ATG CGT GAG ATT gtgagtcctc caacggacgc ccaacgcgtc cctcgcggtgc ccctgatttac c   410
                MET Arg Glu Ile
                 1

411 ccgccgacgg gcgtcgagca acaacattcc tgcccgacac actacccaca gttcgagatg gatcaagaac gtgctgacca tgg   493

494 accttttttt tgttcttgtg atatag GTT CAC CTC CAG ACC GGC CAG TGC gtaagttact tctttccgga cacg          567
                           Val His Leu Gln Thr Gly Gln Cys
                                              10

568 tttgcggtgt gggaggacac agctgatgga cgtgatggaa tag GGC AAC CAG ATT GGT GCT GCT TTC TGG CAG         640
                                             Gly Asn Gln Ile Gly Ala Ala Phe Trp Gln
                                                                              20

641 ACC ATC TCT GGC GAG CAT GGC CTC GAC AGC AAC GGT GTC TAC AAC GGC AGC TCT GAG CTC CAA          703
    Thr Ile Ser Gly Glu His Gly Leu Asp Ser Asn Gly Val Tyr Asn Gly Ser Ser Glu Leu Gln
                          30                                         40

704 CTC GAG CGC ATG AGC GTC TAC TTC AAC GAG gtacgtggatgaactagctacaactgtactgctcgagcagtca         776
    Leu Glu Arg MET Ser Val Tyr Phe Asn Glu
                         50

777 atgctagtgt gggtcctaac aagctgtgca g GCC TCT GGC AAC AAG TAT GTC CCT CGC GCC GTC CTC GTC      846
                                      Ala Ser Gly Asn Lys Tyr Val Pro Arg Ala Val Leu Val
                                                                           60

847 GAT CTT GAG CCC GGT ACC ATG GAC GCT GTT CGT GCG GGT CCT TTC GGC CAG CTC TTC CGC CCC          909
    Asp Leu Glu Pro Gly Thr MET Asp Ala Val Arg Ala Gly Pro Phe Gly Gln Leu Phe Arg Pro
                          70                                         80

910 GAC AAC TTC GTC TTC GGC CAG TCC GGT GCT GGC AAC AAC TGG GCC AAG GGC CAC TAC ACT GAG          972
    Asp Asn Phe Val Phe Gly Gln Ser Gly Ala Gly Asn Asn Trp Ala Lys Gly His Tyr Thr Glu
                          90                                        100

973 GGT GCC GAG CTC GTC GAC AAC GTC CTC GAT GTC GTC CGC CGC GAG GCC GAG GGC TGC GAC TGC        1035
    Gly Ala Glu Leu Val Asp Asn Val Leu Asp Val Val Arg Arg Glu Ala Glu Gly Cys Asp Cys
                         110                                        120

1036 CTC CAG GGC TTC CAG ATC ACC CAC TCC CTG GGT GGT GGC ACT GGT GCC GGT ATG GGC ACC CTG      1098
     Leu Gln Gly Phe Gln Ile Thr His Ser Leu Gly Gly Gly Thr Gly Ala Gly MET Gly Thr Leu
                         130                                       140                      150

1099 CTC ATC TCC AAG ATC CGC GAG GAG TTC CCC GAC CGC ATG ATG GCC ACC TTC TCC GTC GTC CCC      1161
     Leu Ile Ser Lys Ile Arg Glu Glu Phe Pro Asp Arg MET MET Ala Thr Phe Ser Val Val Pro
                                        160                                   170

1162 TCC CCC AAG GTC TCC GAT ACC GTC GTC GAG CCC TAC AAC GCC ACC CTC TCC GTG CAC CAG CTC      1224
     Ser Pro Lys Val Ser Asp Thr Val Val Glu Pro Tyr Asn Ala Thr Leu Ser Val His Gln Leu
                              180                                   190
```

```
1225 GTT GAG CAC TCC GAC GAG ACC TTC TGT ATC GAC AAC GAG GCC CTC TAC GAC ATC TGC ATG CGT    1287
     Val Glu His Ser Asp Glu Thr Phe Cys Ile Asp Asn Glu Ala Leu Tyr Asp Ile Cys MET Arg
                             200                                         210
1288 ACC CTC AAG CTG TCT AAC CCC TCC TAC GGC GAC CTG AAC TAC CTC GTC TCC GCT GTC ATG TCT    1350
     Thr Leu Lys Leu Ser Asn Pro Ser Tyr Gly Asp Leu Asn Tyr Leu Val Ser Ala Val MET Ser
                             220                                         230
1351 GGT GTC ACC ACC TGC CTC CGC TTC CCC GGT CAG CTG AAC TCT GAC CTG CGC AAG CTG GCT GTC    1413
     Gly Val Thr Thr Cys Leu Arg Phe Pro Gly Gln Leu Asn Ser Asp Leu Arg Lys Leu Ala Val
                             240                                         250
1414 AAC ATG GTT CCC TTC CCT CGT CTG CAC TTC TTC ATG GTC GGC TTC GCC CCC CTG ACC AGC CGT    1476
     Asn MET Val Pro Phe Pro Arg Leu His Phe Phe MET Val Gly Phe Ala Pro Leu Thr Ser Arg
                             260                                         270
1477 GGT GCC CAC TCC TTC CGC GCC GTC AGC GTC CCC GAG CTC ACC CAG CAG ATG TTC GAC CCC AAG    1539
     Gly Ala His Ser Phe Arg Ala Val Ser Val Pro Glu Leu Thr Gln Gln MET Phe Asp Pro Lys
                             280                                         290
1540 AAC ATG ATG GCT GCC TCC GAC TTC CGC AAC GGC CGC TAC CTG ACC TGC TCT GCC ATC TT  gta    1601
     Asn MET MET Ala Ala Ser Asp Phe Arg Asn Gly Arg Tyr Leu Thr Cys Ser Ala Ile Ph
                             300                                         310
1602 agttataaag atacgccgag ctctattgtc ttgttccatc tgaagctaac atggaaacag  C CGT GGC AAG GTC GCC   1677
                                                                        e Arg Gly Lys Val Ala
                                                                                320
```

Tab. 2 Teil B

```
1678 ATG AAG GAG GTC GAG GAC CAG ATG CGC AAC GTC CAG AGC AAG AAC TCG TCC TAC TTC GTC GAG    1740
     MET Lys Glu Val Glu Asp Gln MET Arg Asn Val Gln Ser Lys Asn Ser Ser Tyr Phe Val Glu
                             330                                         340
1741 TGG ATC CCC AAC AAC ATC CAG ACC GCT CTC TGC GCC ATT CCT CCC CGT GGC CTC AAG ATG TCC    1803
     Trp Ile Pro Asn Asn Ile Gln Thr Ala Leu Cys Ala Ile Pro Pro Arg Gly Leu Lys MET Ser
                             350                                         360
1804 TCC ACC TTC ATC GGC AAC TCC ACC TCC ATC CAG GAG CTG TTC AAG CGT GTC GGT GAG CAG TTC    1866
     Ser Thr Phe Ile Gly Asn Ser Thr Ser Ile Gln Glu Leu Phe Lys Arg Val Gly Glu Gln Phe
                             370                                         380
1867 ACT GCC ATG TTC CGT CGC AAG GCT TTC CTG CAT TGG TAC ACT GGT GAG GGC ATG GAC GAG ATG    1929
     Thr Ala MET Phe Arg Arg Lys Ala Phe Leu His Trp Tyr Thr Gly Glu Gly MET Asp Glu MET
                             390                                         400
1930 GAG TTT ACC GAG GCC GAG TCC AAC ATG AAC GAC CTC GTC TCC GAG TAC CAG CAG TAC CAG GAT    1992
     Glu Phe Thr Glu Ala Glu Ser Asn MET Asn Asp Leu Val Ser Glu Tyr Gln Gln Tyr Gln Asp
                             410                                         420
1993 GCT GGC ATC GAC GAG GAG GAG GAG GAA TAC GAG GAG GAG CTC CCC CTC GAG GGT GAG GAA TAA    2055
     Ala Gly Ile Asp Glu Glu Glu Glu Glu Tyr Glu Glu Glu Leu Pro Leu Glu Gly Glu Glu TER
                             430                                 440                    447
2056 AAAAAAAAGC TCGCCACCAG AGGCTTGCCG TATACACGGT CCGGCGCGTC GTTCCCGCCA ACTGTGGTAA CCTTTTGAAG TTT   2138

2139 GCAGCCTGTT GCGTTCTATT TATTTCAATT CGGGGTTGTG GAGGTAATTG TGAGAATGGG GTTCTAGA                    2206
```

24

Angaben zu SEQ ID NO: 18

Sequenzkennzeichen:

Länge: 20 Basen

Art: Nukleinsäure mit Aminosäure im kodierenden Bereichen

Strangform: Einzelstrang

Topologie: linear

Ursprüngliche Herkunft:

Organismus: Acremonium chrysogenum

Unmittelbare Herkunft:

Bibliothek: genomisch

Merkmal: Wildtypsequenz als Vergleich zu SEQ ID NO: 7

Sequenzbeschreibung: SEQ ID NO: 18

|  | MET | Ala | Thr | Phe | Ser | Val |  |
|---|-----|-----|-----|-----|-----|-----|---|
| G | ATG | GCC | ACC | TTC | TCC | GTC | G |

**Patentansprüche**

1. β-Tubulin von Acremonium chrysogenum (A. chrysogenum).

2. β-Tubulin nach Anspruch 1 mit der Aminosäuresequenz gemäß Tab. 2 A und B.

3. DNA kodierend für β-Tubulin nach einem der Ansprüche 1 oder 2, oder Teile davon.

4. DNA nach Anspruch 3 mit der Nukleinsäuresequenz gemäß Tab. 2 A und B.

5. DNA nach Anspruch 4, dadurch gekennzeichnet, daß sie aus dem proteinkodierenden Bereich gemäß Tab. 2 A und B besteht.

6. DNA kodierend für β-Tubulin oder Teile davon, dadurch gekennzeichnet, daß sie unter stringenten Bedingungen zu dem proteinkodierenden Bereich der DNA-Sequenz gemäß Tab. 2 A und B oder Teile davon hybridisiert.

7. DNA nach Anspruch 3, dadurch gekennzeichnet, daß die Aminosäure Nummer 167 Tyrosin ist.

8. Vektor enthaltend eine DNA nach mindestens einem der Ansprüche 3 bis 7.

9. Vektor nach Anspruch 8, dadurch gekennzeichnet, daß es der Vektor pAUL1 gemäß Fig. 5 ist.

**10.** Wirtszelle enthaltend eine DNA nach mindestens einem der Ansprüche 3 bis 7 oder einen Vektor nach Anspruch 8 oder 9.

**11.** Verfahren zur Herstellung von β-Tubulin nach Anspruch 1 oder 2, dadurch gekennzeichnet, daß
    a) eine Wirtszelle nach Anspruch 10 kultiviert
    und
    b) β-Tubulin isoliert wird.

**12.** Verfahren zur Transformation von A. chrysogenum, dadurch gekennzeichnet, daß A. chrysogenum mit DNA nach mindestens einer der Ansprüche 3 bis 7 oder mit einem Vektor nach einem der Ansprüche 8 oder 9 in Kontakt gebracht wird.

**13.** Verfahren nach Anspruch 12, dadurch gekennzeichnet, daß vor der Transformation Protoplasten von A. chrysogenum hergestellt werden und die transformierten Protoplasten in einem geeignetem Medium regeneriert werden.

**14.** Verfahren nach Anspruch 13, dadurch gekennzeichnet, daß das Medium 11 bis 50 Gew.-% Saccharose enthält.

**15.** Verfahren zur Kotransformation von A. chrysogenum, dadurch gekennzeichnet, daß A. chrysogenum mit DNA nach mindestens einen der Ansprüche 3 bis 7 oder mit einem Vektor nach einem der Ansprüche 8 oder 9 zusammen mit einer DNA, die kotransformiert werden soll, in Kontakt gebracht wird.

**16.** Verfahren nach Anspruch 15, dadurch gekennzeichnet, daß die verwendeten DNAs oder Vektoren ausschließlich aus zu A. chrysogenum homologen DNA-Sequenzen bestehen.

**17.** Verfahren zur Herstellung von Fremproteinen in A. chrysogenum, dadurch gekennzeichnet, daß A. chrysogenum verwendet wird, das mit einer DNA nach mindestens einem der Ansprüche 3 bis 7 oder mit einem Vektor nach einem der Ansprüche 8 oder 9 und einer DNA, die für das gewünschte Fremdprotein kodiert, kotransformiert wurde.

**18.** Verwendung einer DNA nach mindestens einem der Ansprüche 3 bis 7 oder eines Vektors nach Anspruch 8 oder 9 zur Transformation von A. chrysogenum.

Fig. 1

EP 0 610 842 A2

_Fig. 2_

*Fig. 3*

EP 0 610 842 A2

**Fig. 4**

| IVS: | 1 | 2 | 3 | 4 | 5 | | 6 | | β-TUBULIN |
|------|---|---|---|---|---|---|---|---|---|
| Ac | + | + | − | − | + | | + | | |
| Nc | + | + | + | + | + | | + | | |
| An | + | + | + | + | + | + (AA 205-206) | + | + (AA 437-438) | |
| Cg | + | + | + | + | + | | +[1] | | |
| Cga | + | + | + | − | − | | − | | |
| Sp | + | − | + | + | + | | − | + (AA 349) | |
| Sc | − | − | − | − | − | | − | | |

POSITION:  4-5   21  35        317
          12-13      53-54

Fig. 5